# EUROPEAN PATENT SPECIFICATION

(11) **EP 1 072 189 B1**
(45) Date of publication and mention of the grant of the patent: **18.12.2002**
(21) Application number: 00202573.2
(22) Date of filing: 18.07.2000
(51) Int. Cl.: A01K 67/033

(54) **Device for breeding snails**
Vorrichtung zur Schneckenzucht
Dispositif pour élever des escargots

(30) Priority: 30.07.1999 NL 1012746
(43) Date of publication of application: 31.01.2001
(73) Proprietor: Coenen, Henricus Petrus Wilhelmus, 6095 EK Baexem (NL)
(72) Inventor: Coenen, Henricus Petrus Wilhelmus, 6095 EK Baexem (NL)
(74) Representative: Valkonet, Rutger

(56) References cited:
- BE-A- 1 006 758
- DE-U- 29 701 313
- FR-A- 2 623 689
- US-A- 3 111 915
- US-A- 5 779 068

## Description

The invention relates to a device for breeding snails built up in the form of an open frame construction, consisting of an undersurface assembled from girders and posts placed on said surface, wherein several plate-shaped breeding surfaces can be accommodated between said posts.

Snails, and in particular escargots, are known as a culinary delicacy. The breeding of snails for human consumption takes place in particular in South-European countries. The snails are thereby kept in their natural surroundings, for example parks, which are completely screened off by means of nets in order to keep the snails inside and their natural enemies outside the breeding area.

A breeding method of this kind has a large number of drawbacks. First of all, the weather conditions have a great influence on the development and the survival of the snail population, in particular ground frost may have disastrous consequences for the snails. In addition to that, the snails are susceptible to harmful fungi and parasites, which leads to undesirable losses. Furthermore, snails of various generations, size and quality will develop in a breeding area of this kind, as a result of which the yield will be uneconomic. Moreover, the harvesting of the snails is a time-consuming and laborious activity when this method is used.

An example of a breeding device according the preamble of claim 1 is for example known from the Belgian patent publication BE-A-1006758. In that publication a frame construction is disclosed forming compartments for keeping snails, which compartments are closed by means of a metal grating plates. However such construction has several drawbacks: although the grating plates prevent the snails from migrating the device, the grating plates need to be cleaned during regular intervals as they are susceptible to clogging with impurities from the snails. Furthermore for feeding, maintenance and other purposes the grating plates need to be opened by hand, allowing the snails present on said grating to escape, which makes the device less user friendly.

The object of the invention is to overcome the above drawbacks and to provide a breeding device for snails by means of which large amounts of snails of the same age, size and quality can be bred and harvested in a labour-friendly manner and with minimal losses. This object is achieved by a device according to claim 1.

The device is according to the invention characterized in that two wires extending parallel to each other are fitted in the circumferential direction of an open side of said frame construction, between which wires a voltage difference is applicable.

In German Gebrauchsmuster DE-U-29701313 a snail trap is disclosed using two wires through which an electrical current is applied. However no reference is made to a device for breeding snails in can industrial way.

According to the invention, in order to stimulate the snails to move around and also to assist in the removal of faeces and other impurities, the breeding surfaces can be accommodated in the frame in such manner as to slope down towards one side of the frame.

In order to prevent the snails migrating from the device, the posts are provided with a large number of evenly spaced bores in one embodiment of the device, into which bores supporting hooks can be inserted, which hooks support the plate-shaped breeding surfaces near their corner points.

In order to be able to create advantageous climate conditions in the device, said undersurface and said upper surface as well as the open sides of the frame can be screened off by means of plates in one embodiment.

Near the upper surface, said wires can be interconnected with corresponding wires of a further breeding device according to the invention.

In one embodiment of the device, strips may be mounted along the posts and the lowermost girder on the open side, to which strips said wires are attached.

In order to enable a more flexible use of the breeding device according to the invention, the device can be moved on wheels mounted on the undersurface.

The breeding device according to the invention will now be explained in more detail with reference to a drawing, which drawing successively shows in:
Figure 1 an embodiment of a breeding device according to the invention;
Figures 2a - 2c partial aspects of the embodiment of Figure 1.

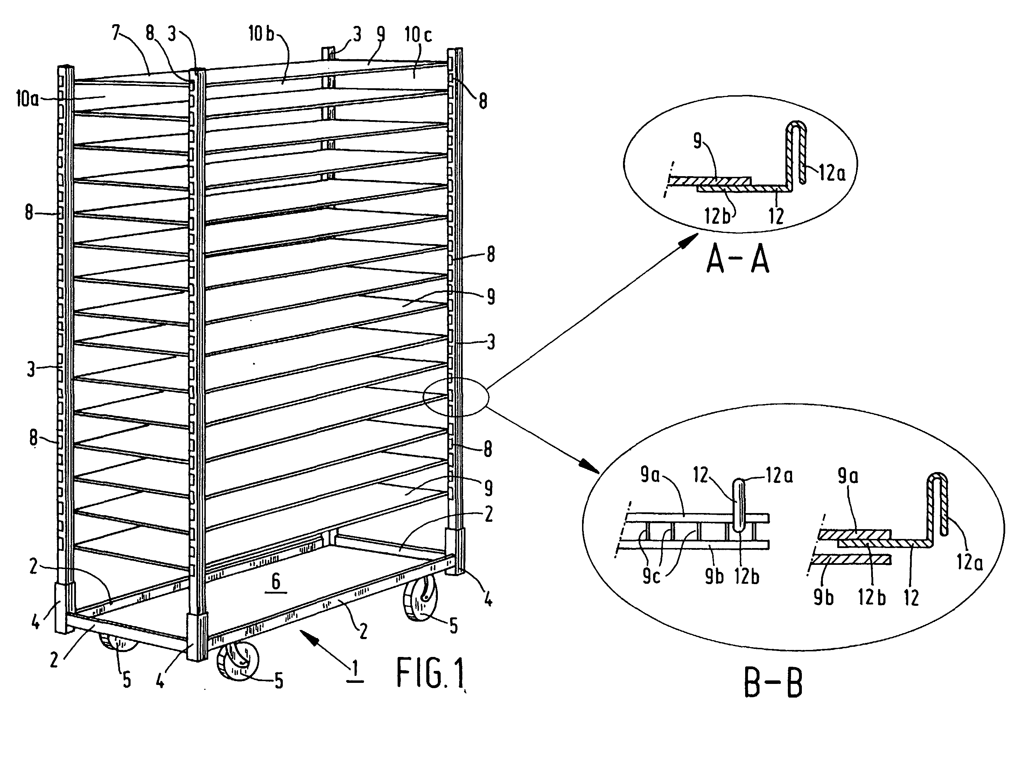

Figure 1 shows the breeding device according to the invention. The device comprises an open frame construction consisting of a chassis 1 assembled from girders 2, on which hollow pipe members 4 are disposed. Four posts 3 extend into said pipe members 4. Chassis 1 is fitted with wheels 5, as a result of which the entire device can easily be moved. The bottom and upper sides of the frame of the device can be screened off by means of plates 6 and 7, respectively.

The posts 3 are provided with a large number of equally spaced openings 8. Supporting hooks, on which plates 9 are supported, can be inserted into said openings 8. In this manner a large number of plates 9 can be accommodated in the open frame construction (chassis 1, girders 2 and posts 3). Preferably, plates 9 slope down slightly towards one side of the frame.

The snails, preferably of the same generation, which are still young at that stage, are put out on plates 9. The population of snails thus put out all have the same age and will be the same size and of the same quality at the time of being harvested. This will result in a very uniform and profitable yield.

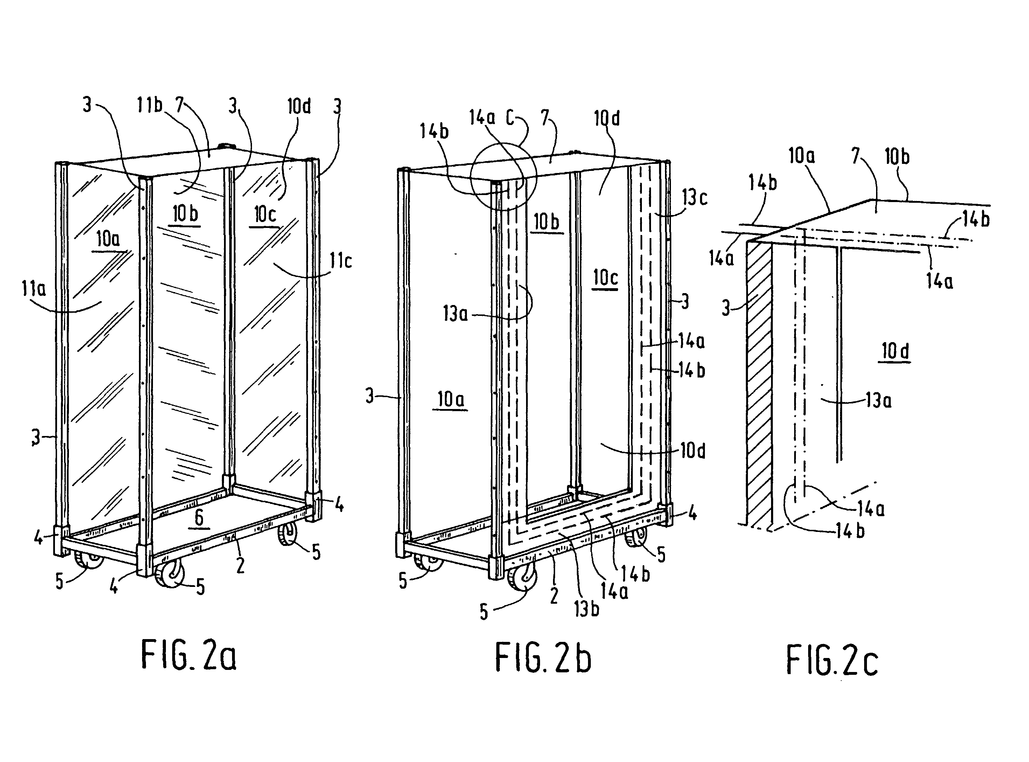

The open sides 10a-10c can also be screened off by means of plates 11a-11c, which may or may not be transparent. This construction is shown in Figure 2a, from which the breeding plates 9 have been left out for the sake of clarity. The transparent yet screened-off and lightweight frame construction offers a good view of the population of snails put out on plates 9. Any sick and/or dead snails can easily be removed via the front, open side 10d. The screening plates 11a-11c prevent the snails from migrating.

An ideal control of the climate conditions can be achieved by placing the breeding device in a cell as used in mushroom culture. Such a cell makes it possible to create and control ideal temperature and humidity conditions, so that the snails can thrive optimally. In particular in a mushroom cell it is possible to maintain a constant temperature of 20°C - 22°C, which is an ideal temperature for snails. In other types of propagating houses the temperature fluctuates too much, which is disadvantageous for the development of the snail population. When temperatures are too high, the snails will start their "summer sleep", as a result of which they will stop growing. In such an isolated cell, the population cannot be endangered by parasites or fungi. This will reduce losses and result in a good yield, both as regards quantity and as regards quality.

The advantage of the construction is that a large population of snails, all of the same generation, are housed in one compact space. Of course the density of the snail population must be such that the snails will have sufficient space to move about. In order to stimulate the snails to move about, the breeding plates 9 are disposed slightly obliquely in the frame construction, whereby they preferably slope down towards the rear side 10b. The additional advantage of this is that the breeding plates are easy to hose down: faeces and other impurities are washed away towards the rear side 10b.

Two measures prevent the snails escaping from the breeding device. The first measure is shown in the enlarged detail A-A of Figure 1, it concerns the manner in which the breeding plates 9 are mounted between posts 3 in the frame construction. Each breeding plate 9 is supported by supporting hooks 12 near its corner points, which supporting hooks include a bent-over end portion 12a. The bent-over end portion 12a can be inserted into one of many openings 8 present in post 3. Hook 12 is preferably made of an elongated, thin strip or of a thin yet rigid bar, for example made of a plastic or a metal.

As a result of this manner of mounting, the breeding plates are disposed completely clear of the four posts 3 in the device, without coming into contact with screening plates 11a-11c. The breeding plate is furthermore supported on four supporting hooks, with a very narrow transition being present between the breeding plate and the post 3. Thus the snails cannot migrate directly from the breeding plates onto the screening plates 11a-11c, nor will they be able to cross the narrow transition between the breeding plate 9 and the post 3 of the supporting hook 12.

The breeding plate 9 may also consist of an upper plate 9a and a lower plate 9b, which are held some distance apart by means of regularly spaced-apart cross connections 9c (see enlarged detail B-B of Figure 1). The end 12b of supporting hook 12 can thus be inserted into the open "sandwich" construction of breeding plate 9, so that said plate is suspended between posts 4. This makes it possible to accommodate the breeding plates 12 in a downwardly sloping position in the frame construction without said breeding plates sliding off the supporting hooks 12.

A further measure to prevent snails migrating and/or escaping from the breeding device is shown in Figures 2b and 2c. Two parallel wires 14a and 14b are thereby used, between which wires a voltage difference can be applied. The magnitude of the voltage being applied depends on the age of the snails, among other things. To this end, transparent strips of material 13a-13c are fitted in the plane of the open front side 10d, along and against posts 3 and girder 2. The two wires 14a and 14b are present on the inwardly facing side of said strips 13a-13c and on the underside of upper plate 7. Wires 14a-14b each form a closed loop.

In the unwished-for case that a snail should move off a breeding plate and land on the inner side of one of the strips 13a-13c or on the underside of upper plate 7, wires 14a and 14b will prevent the snail leaving the breeding device. If the snail comes into contact with both wires, a light current impulse will cause the snail to move back. The required voltage can be supplied by a battery present on upper plate 7, for example. The use of a transformer is to be preferred, however: a transformer makes it possible in a simple manner to regulate the required voltage in dependence on the age of the snails.

Possibly, the two electric wires 14a and 14b can be interconnected with the two corresponding electric wires 14a and 14b of another breeding device, as already described before. When said electric screens are interconnected, only one voltage source is required. In addition, several breeding devices disposed side by side enable large-scale breeding of snails, wherein each device accommodates a population of snails of a particular age. This ensures a continuous yield of snails throughout the year, especially if the breeding devices are housed in mushroom farms, so that ideal breeding conditions (temperature, humidity) can be realised.

It will be apparent that large amounts of snails of the same generation, size and quality can be bred in a simple manner by means of the present breeding device. Snails losses due to diseases, fungi or parasites are minimized, especially if ideal climate conditions can be realised by housing the breeding devices in mushroom farms. The breeding of snails by means of a breeding device according to the invention is furthermore very labour-friendly.

## Claims

1. A device for breeding snails, built up in the form of an open frame construction, consisting of an undersurface (1) assembled from girders (2) and posts (3) placed on said surface (1), wherein several plate-shaped breeding surfaces (9) can be accommodated between said posts (3), **characterized in that** two wires (14a, 14b) extending parallel to each other are fitted in the circumferential direction of an open side (10d) of said frame construction, between which wires (14a, 14b) a voltage difference is applicable.

2. A breeding device according to claim 1, **characterized in that** the breeding surfaces (9) are accommodated in the frame in such manner as to slope down towards one side of the frame.

3. A breeding device according to claim 1 or 2, **characterized in that** the posts (3) are provided with a large number of evenly spaced bores (8), into which supporting hooks (12) are inserted, which hooks support the plate-shaped breeding surfaces (9) near their corner points.

4. A breeding device according to any one of the preceding claims, **characterized in that** said undersurface (6) and said upper surface (7) as well 1 as the open sides of the frame are screened off by means of plates (11a-11c).

5. A breeding device according to anyone of the preceeding claims, **characterized in that** said wires (14a, 14b) are interconnected with corresponding wires of a further breeding device according to the invention near the upper surface (7).

6. A breeding device according to anyone of the preceding claims, **characterized in that** strips (13a-13c) are mounted along the posts (3) and the lowermost girder (2) on the open side (10d), to which strips said wires (14a, 14b) are attached.

7. A breeding device according to any one of the preceding claims, **characterized in that** the device is movable on wheels (5) mounted on the undersurface.

## Patentansprüche

1. Vorrichtung zur Schneckenzucht in Form einer offenen Rahmenkonstruktion, bestehend aus einer aus Trägern (2) zusammengebauten Unterseite (1) und auf der Seite (1) angeordneten Tragstützen (3), wobei mehrere plattenförmige Zuchtflächen (9) zwischen den Tragstützen (3) gelagert werden können, **dadurch gekennzeichnet, dass** zwei, sich parallel zueinander erstreckende Drähte (14a, 14b) in Umfangsrichtung einer offenen Seite (10d) der Rahmenkonstruktion angeordnet sind, wobei zwischen diesen Drähten (14a, 14b) eine Spannungsdifferenz angelegt werden kann.

2. Zuchtvorrichtung gemäß Anspruch 1, **dadurch gekennzeichnet, dass** die Zuchtflächen (9) in dem Rahmen derart angeordnet sind, dass sie sich zu einer Seite des Rahmens hin nach unten abschrägen.

3. Zuchtvorrichtung gemäß Anspruch 1 oder 2, **dadurch gekennzeichnet, dass** die Tragstützen (3) mit einer großen Anzahl gleichmäßig voneinander beabstandeter Bohrungen (8) versehen sind, in welche Traghaken (12) eingeschoben werden, wobei die Haken die plattenförmigen Zuchtflächen (9) in der Nähe ihrer Eckpunkte tragen.

4. Zuchtvorrichtung gemäß irgendeinem der vorherigen Ansprüche, **dadurch gekennzeichnet, dass** die Unterseite (6) und die Oberseite (7) sowie die offenen Seiten des Rahmens anhand von Platten (11a-11c) abgeschirmt sind.

5. Zuchtvorrichtung gemäß irgendeinem der vorherigen Ansprüche, **dadurch gekennzeichnet, dass** die Drähte (14a, 14b) mit entsprechenden Drähten einer weiteren erfindungsgemäßen Zuchtvorrichtung nahe der Oberseite (7) verbunden sind.

6. Zuchtvorrichtung gemäß irgendeinem der vorherigen Ansprüche, **dadurch gekennzeichnet, dass** Leisten (13a-13c) entlang der Tragstützen (3) und des untersten Trägers (2) an der offenen Seite (10d) angebracht sind, wobei an diesen Leisten die Drähte (14a, 14b) befestigt sind.

7. Zuchtvorrichtung gemäß irgendeinem der vorherigen Ansprüche, **dadurch gekennzeichnet, dass** die Vorrichtung auf an der Unterseite angebrachten Rädern (5) beweglich ist.

## Revendications

1. Un dispositif pour élever des escargots, réalisé à la forme d'une construction à cadre ouvert, formé d'une surface inférieure (1) assemblée à partir de poutrelles (2) et de piliers (3) placés sur ladite surface (1), dans lequel une pluralité de surfaces d'élevage (9) en forme de plaque peuvent être logées entre lesdits piliers (3), **caractérisé en ce que** deux fils (14a, 14b), s'étendant parallèlement l'un à l'autre, sont montés en direction circonférentielle d'un côté ouvert (10d) de ladite construction en cadre, fils (14a, 14b) entre lesquels peut être appliquée une différence de tension.

2. Un dispositif d'élevage selon la revendication 1, **caractérisé en ce que** les surfaces d'élevage (2) sont logées dans le cadre, de manière à être inclinées vers le bas sur un côté du cadre.

3. Un dispositif d'élevage selon la revendication 1 ou 2, **caractérisé en ce que** les piliers (3) sont pourvus d'un grand nombre de trous (8) uniformément espacés, dans lesquels sont insérés des crochets supports (12), les crochets supportant les surfaces d'élevage (9) en forme de plaque, à proximité de leurs angles.

4. Un dispositif d'élevage selon l'une quelconque des revendications précédentes, **caractérisé en ce que** ladite surface inférieure (6) et ladite surface supérieure (7) ainsi que les cotés ouverts du cadre sont isolées à l'aide de plaques (11a à 11c).

5. Un dispositif d'élevage selon l'une quelconque des revendications précédentes, **caractérisé en ce que** lesdits fils (14a, 14b) sont interconnectés à des fils correspondants d'un autre dispositif d'élevage selon l'invention, à proximité de la surface supérieure (7).

6. Un dispositif d'élevage selon l'une quelconque des revendications précédentes, **caractérisé en ce que** des bandes (13a à 13c) sont montées sur les piliers (3) et la poutrelle à la position la plus basse sur le côté ouvert (10d), bandes sur lesquelles sont fixés lesdits fils (14a, 14b).

7. Un dispositif d'élevage selon l'une quelconque des revendications précédentes, **caractérisé en ce que** le dispositif est déplaçable sur des roues (5) montées sur la surface inférieure.
